# EUROPEAN PATENT APPLICATION

(11) **EP 1 576 955 A1**
(43) Date of publication of application: **21.09.2005**
(21) Application number: 05075389.6
(22) Date of filing: 17.02.2005
(51) Int. Cl.: A61K 31/45, A61K 31/445, A61K 31/473

(54) **Combinatorial therapy with an acetylcholinesterase inhibitor and (3aR) 1,3a,8 trimethyl 1,2,3,3a,8,8a hexahydropyrrolo[2,3 b]indol 5 yl phenylcarbamate**

(30) Priority: 17.02.2004 US 545046 P
(71) Applicant: Axonyx, Inc., New York, NY 10018 (US)
(72) Inventor: Bruinsma, Gosse B., 2312 TT Leiden (NL)
(74) Representative: Winckels, Johannes Hubertus F.

(57) **Abstract**

The present invention relates to an improved method of treating, reducing or delaying cognitive impairments associated with the β-amyloid peptide, either in combination with treatment by cholinomimetic replacement therapy or as a prophylactic treatment.

## Description

### CROSS-REFERENCE TO RELATED APPLICATIONS

This application claims the benefit of U.S. Patent Application 60/545,046, filed February 17, 2004, the entirety of which is incorporated by reference.

### TECHNICAL FIELD

The invention relates to generally to biotechnology, and more particularly to various methods and compositions for the treatment of cognitive disorders.

### BACKGROUND

Since defects in the cholinergic system have been suggested to underlie cognitive impairments associated with normal aging and Alzheimer's disease (AD) (Bartus *et al., Science* 217:408-417 (1982); Fischer *et al., Neurobiol. Aging* 13:9-23 (1992)), much research has focused on the development of cholinomimetic replacement therapy as a potential treatment of these impairments. Among them, cholinesterase inhibitors, such as physostigmine (Phy) and tetrahydroaminoacridine (THA), have been investigated for memory-enhancing effects in both animals (Rupniak *et al., Neurobiol. Aging* 11:09-613 (1990); Murray *et al. Psychopharmacology* 105:134-136 (1991)) and human subjects (Mohs *et al., J A. Geriatr. Soc.* 33:749-757 (1985); Summers *et al., N. Engl. J. Med.* 315:1241-1245 (1986)).

Additional cholinesterase inhibitors that are, or have been, used for treating cognitive disorders, such as Alzheimer's disease, included donepezil, rivastigmine, and galantamine, *see,* U.S. Patent 5,409,948. In addition, (-)-phenserine, the negative optical enantiomer (-)-phenylcarbamoyleseroline, which has the structure: 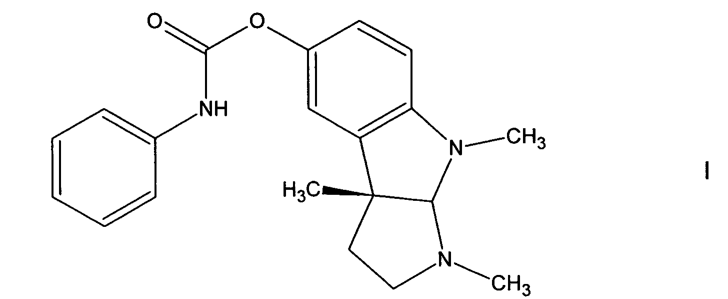 (3a*S*)-1,3a,8-trimethyl-1,2,3,3a,8,8a-hexahydropyrrolo[2,3-*b*]indol-5-yl phenylcarbamate
and its salts, provides another acetylcholinesterase inhibitor that may be used clinically for treating cognitive disorders.

(-)-Phenserine is a potent acetylcholinesterase inhibitor having significantly reduced negative side effects relative to other acetylcholinesterase inhibitors. Due to the chronic nature of treatment for cognitive disorders, it has long been desired to provide an agent that is effective and does not produce the undesirable side effects that may be caused by the use of acetylcholinesterase inhibitors.

Many of the drugs currently available for the treatment of cognitive impairments, such as Alzheimer's Disease, are prepared as a chemically pure enantiomer. For example, rivastigmine, and galantamine are both prepared as the desired enantiomer, and considerable attention is paid to avoiding the alternative enantiomer or purifying the desired enantiomer. This resolution of enantiomers substantially increases the cost of manufacturing the drug.

(-)-Phenserine, the (-) enantiomer, was initially prepared as the pure (-) enantiomer for use in the treatment of cognitive impairments, since this enantiomer inhibits acetylcholinesterase activity, while the (+) enantiomer, POSIPHEN™, does not.

### DISCLOSURE OF INVENTION

The present invention provides combinatorial therapy for the treatment of cognitive disorders, such as those associated with accumulation of a β-amyloid peptide.

The present invention relates to an improved method of therapy for cognitive impairments, comprising treating a subject with an effective amount of a compound lacking significant cholinergic activity, but having β-APP or Aβ inhibitory activity (which may be determined, for example, by the method described in Examples II and/or V), and a compound having cholinergic activity, for example, (-) and (+)-N-phenylcarbamoyl eseroline, and/or one or more pharmaceutically acceptable salts of each. In particular, cognitive disorders associated with the β-amyloid peptide production and/or accumulation and susceptible to treatment by cholinomimetic replacement therapy may be treated.

The present invention relates to an improved method of treating cognitive disorders comprising treating a subject with an effective amount of an acetylcholinesterase inhibitor (AChEI) and a compound lacking significant AChEI activity, but being capable of reducing the production of β-APP and/or accumulation of Aβ, for example, (+)-phenserine, POSIPHEN™. In particular, cognitive disorders associated with the β-amyloid peptide and susceptible to treatment by cholinomimetic replacement therapy may be benefited treated by the method of the invention.

The invention further relates to co-administration of (+)-phenserine or a similar compound and at least one acetylcholinesterase inhibitor selected from the group consisting of (-)-phenserine, donepezil, rivastigmine, metrifonate, tacrine, physostigmine, (-) carbamates, eptastigmine, galantamine, huperzine A and pharmaceutically acceptable salts and esters thereof.

The invention further relates to coadministration of (+)-phenserine and an AchEI for the symptomatic treatment of AD.

In a preferred embodiment, the invention further relates to the coadministration of (+)-phenserine and (-)-phenserine, preferably in non-equal molar amounts, including between about 2:1 to about 100:1.

The invention further relates to a method of reducing the subject's risk of developing or further developing a cognitive impairment and/or providing a prophylactic treatment for subject comprising co-administering (+)-phenserine to a subject thought to be at risk for the development of a cognitive impairment associated with increased levels of β-APP or Aβ, for example, in combination with an AChE inhibitor.

Typically, treatment of cognitive disorders associated with accumulation of Aβ, such as Alzheimer's disease, are initiated when the subject's cognitive impairment has progressed to a point where cognitive ability displays significant impairment that can be attributed to a disease state, such as Alzheimer's disease. The fact that many of the subjects are elderly and may be simply suffering from other effects of aging hinders the diagnosis of AD and delays treatment. The present invention overcomes this difficulty and provides a method of treating an elderly subject or a subject having a perceived risk of developing a cognitive impairment without the necessity of awaiting manifestations of actual cognitive decline.

The present invention also relates to co-administration of effective amounts of N-phenylcarbamoyl eseroline enantiomers, or a pharmaceutically acceptable salt of one or more enantiomers. In an exemplary embodiment, the (-) enantiomer is administered at a dosage appropriate for cholinomimetic replacement therapy and the (+) enantiomer is administered at a dosage appropriate for the reduction or further reduction of β-APP synthesis when co-administered with the (-) enantiomer.

The present invention also relates to co-administration of an effective amount of(+) and (-) enantiomers of (-)-phenserine, or a pharmaceutically acceptable salt of the enantiomers. In an exemplary embodiment, the (-)-phenserine is administered at a dosage appropriate for cholinomimetic replacement therapy and (+)-phenserine is administered at a dosage appropriate for the reduction or further reduction of β-APP synthesis when co-administered with an AChEI, such as (-)-phenserine.

The invention further relates to a method of treating a subject thought to be in need of such treatment comprising administering an effective amount of a mixture of N-phenylcarbamoyl eseroline enantiomers according to the present invention to the subject, e.g., a mammal, such as a human.

The present invention also relates to the use of a composition containing (+)-phenserine tartrate with an acetylcholinesterase inhibitor, such as (-)-phenserine tartrate. In addition, the invention relates to the use of a pharmaceutical composition containing a pharmaceutical (+)-phenserine tartrate salt and an acetylcholinesterase inhibitor, such as a (-)-phenserine tartrate salt.

In a preferred embodiment, the invention further relates to a method of manufacturing a pharmaceutical composition comprising an AChEI and (+)-phenserine, or a pharmaceutically acceptable salt thereof, for the treatment of cognitive disorders, such as Alzheimer's disease, vascular dementia and other disease states associated with the β-amyloid peptide and susceptible to treatment by cholinomimetic replacement therapy. In an exemplary embodiment, the invention relates to the use of (+)-phenserine or a pharmaceutically acceptable salt or ester thereof in combination with an AChEI selected from the group consisting of at least one of donepezil, metrifonate, tacrine, flurbiprofen, and rivastigmine.

### BRIEF DESCRIPTION OF DRAWINGS

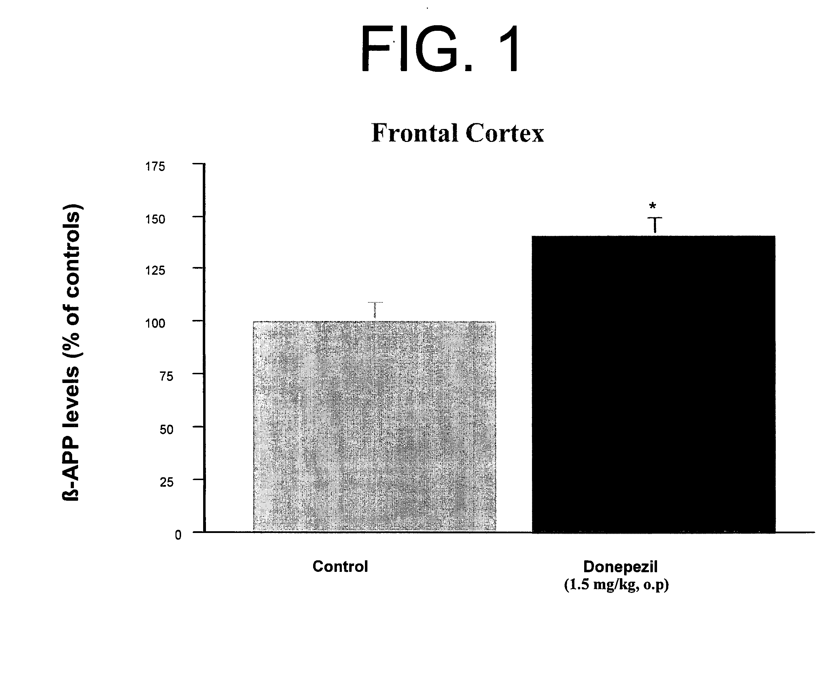

FIG. 1 illustrates the effect of three weeks of daily oral administration of donepezil (1.5 mg/kg) on brain levels of β-APP in adult rats (N>6). The levels of β-APP were measured by 22C11 (epitope APP 66-81). Similar elevations were seen with Elan 266 (epitope Aβ 13-28) for β-APPγ. * Significantly different from controls (p<0.05).

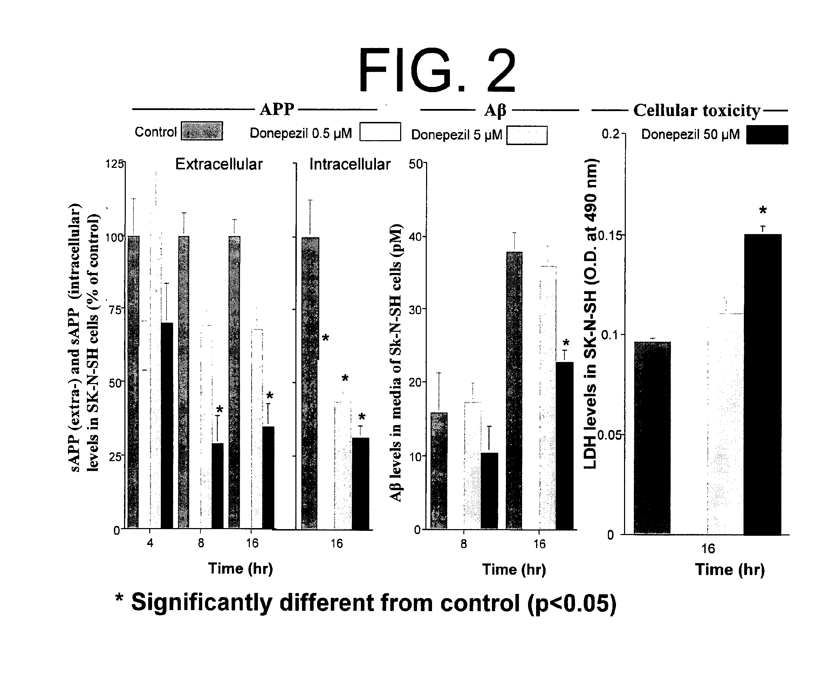

FIG. 2 illustrates the effect of donepezil treatment on β-APP and Aβ levels. Donepezil reduces intracellular β-APP without altering Aβ levels, except at toxic doses, as measured by LDH.

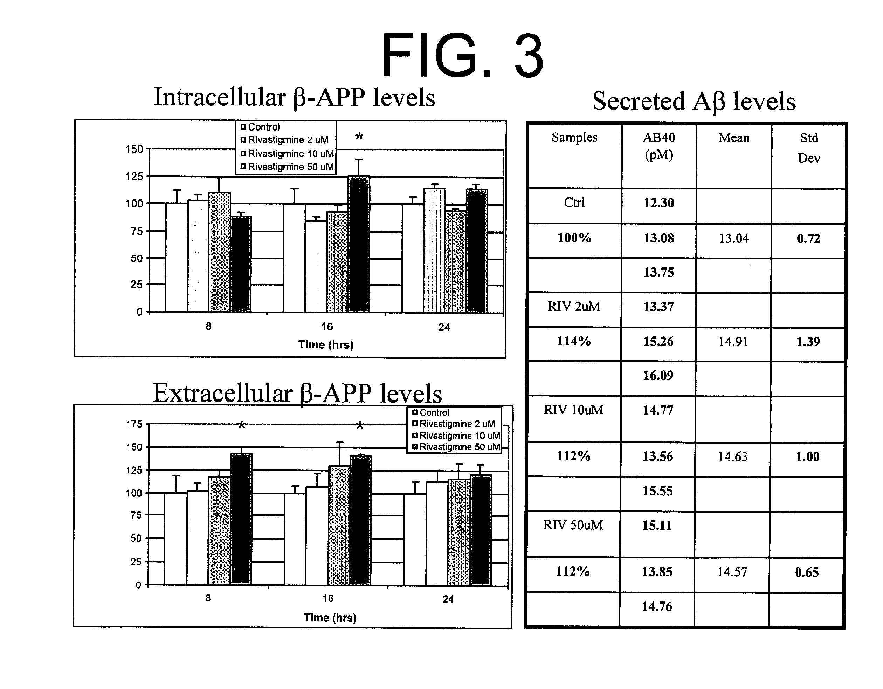

FIG. 3 illustrates the elevated secretion of β-APP by rivastigmine in human neuroblastoma cells. Human neuroblastoma cells were incubated with rivastigmine at 2 µM, 10 µM and 50 µM, and secreted β-APP and Aβ were measured. No statistically significant change in the Aβ peptide levels was seen.

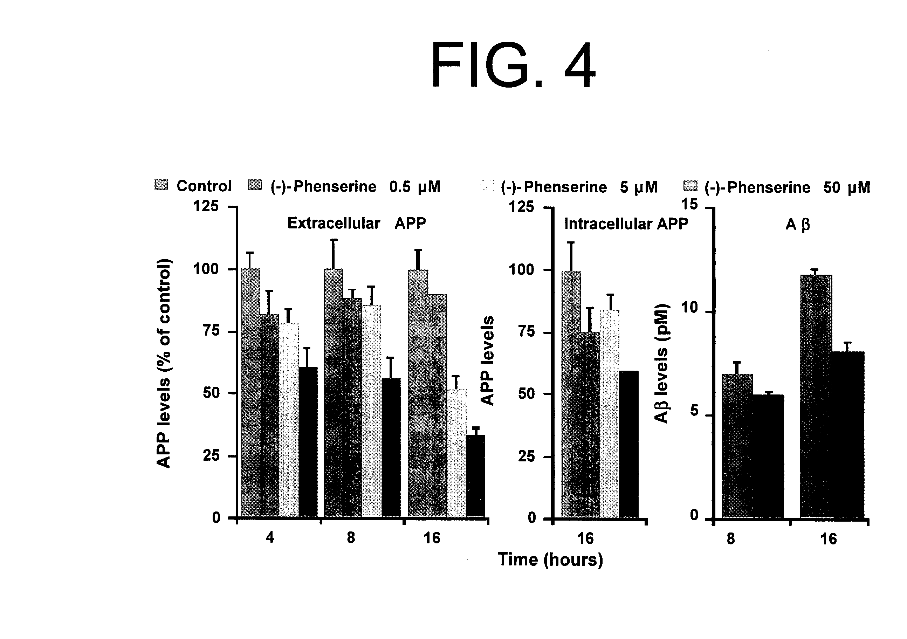

FIG. 4 illustrates time- and concentration-dependent actions of (-)-phenserine on cellular (intracellular) and secreted (extracellular) levels of β-APP and secreted levels of Aβ in human neuroblastoma (SK-N-SH) cell culture, as assessed by Western blot analysis of β-APP levels (mAb 22C11) and ELISA analysis of Aβ in the absence of cellular toxicity, as assessed by measurement of lactase dehydrogenase levels. (-)-phenserine significantly lowered β-APP and Aβ levels (p<0.05 Dunnett's T-test).

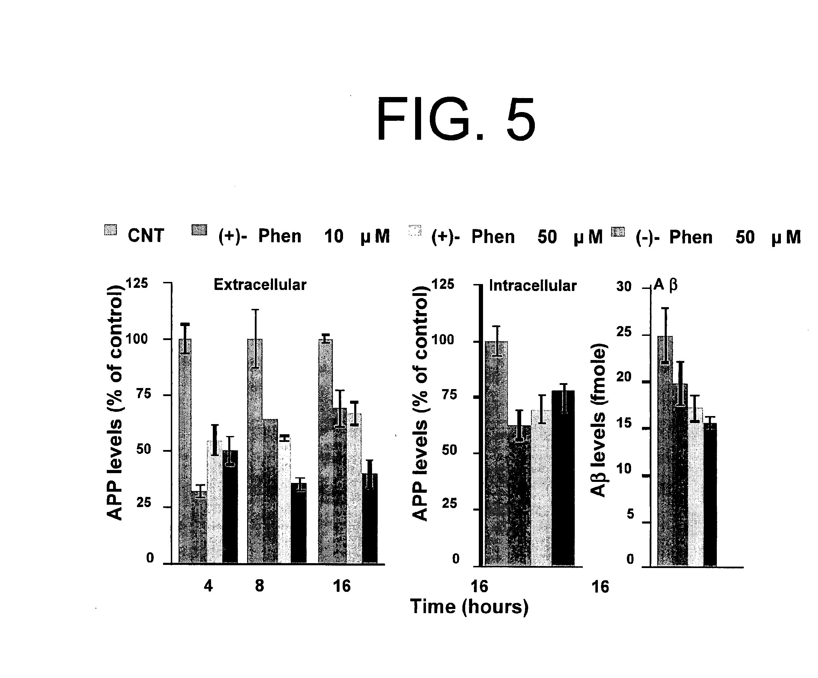

FIG. 5 illustrates time- and concentration-dependent actions of (+)-phenserine ((3aR)-1,3a,8-trimethyl-1,2,3,3a,8,8a-hexahydropyrrolo[2,3-b]indol -5-yl phenylcarbamate) compared to (-)-phenserine on intracellular and extracellular levels of β-APP and secreted levels of Aβ in human neuroblastoma (SK-N-SH) cell culture, as assessed by Western blot analysis of APP levels (mAb 22C11) and ELISA analysis of Aβ in the absence of cellular toxicity, as assessed by measurement of lactase dehydrogenase levels. Both (-)-phenserine and (+)-phenserine significantly lowered β-APP and Aβ levels (p<0.05 Dunnett's T-test).

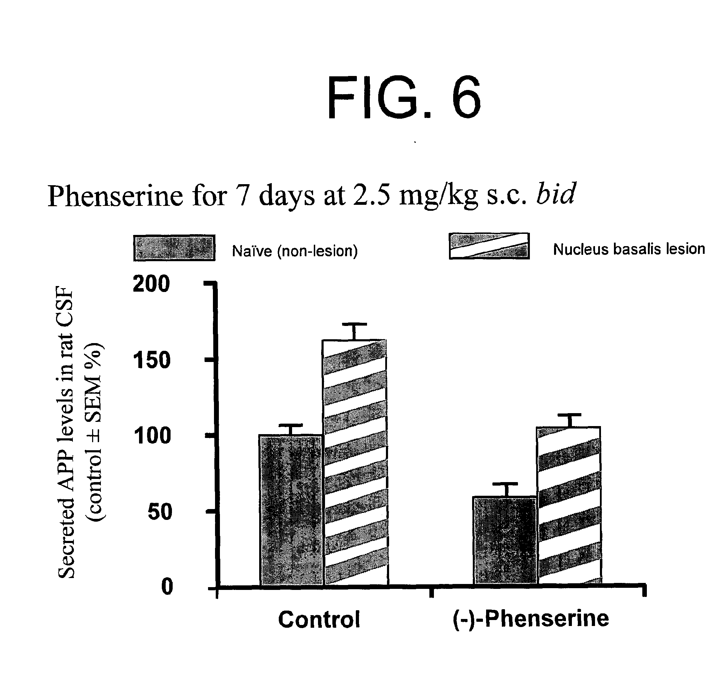

FIG. 6 illustrates the action of (-)-phenserine on CSF levels of β-APP in naïve rats and animals with cholinergic forebrain (unilateral neurotoxic nucleus basalis of Meynert) lesions. (-)-phenserine significantly (p<0.05, N=7-8 rats per group) lowered β-APP levels in naïve and lesion animals, compared to untreated controls (p<0.05). Lesions were induced with 1 ml N-methyl-D-aspartic acid (50 nMol) (64-68).

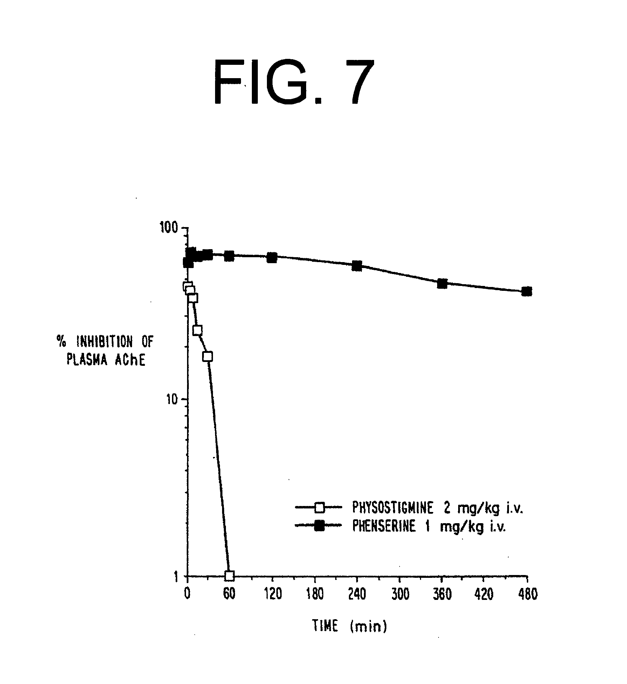

FIG. 7 illustrates the *in vivo* AChE inhibition by i.v. administration of physostinine and (-)-phenserine in serial samples of plasma from Fisher-344 rats.

### BEST MODE(S) FOR CARRYING OUT THE INVENTION

In accordance with this invention, the compound of formula II, (+)-phenserine, which includes pharmaceutically acceptable salts and esters thereof, is effective for treating subjects suffering from cognitive disorders or preventing the development or progression thereof. In addition, administering (+)-phenserine or its pharmaceutically acceptable salts or esters in combination with the compound of formula I ((-)-phenserine) may be effective for treating subjects suffering from cognitive disorders. This effect is especially surprising in view of the fact that the compound of the formula II, which is (+) 9 -N- phenylcarbinol esroline, the non-natural (+) isomer of (-)-phenserine, unlike (-)-phenserine, has minimal or no anticholinesterase activity. Therefore, the (+) and (-) isomers have unrelated mechanisms of action with respect to anticholinesterase activity (*see,* PCT International publication WO 03/082270, incorporated herein by reference). Particularly, (-)-phenserine is an anticholinesterase inhibitor and (+)-phenserine is not. 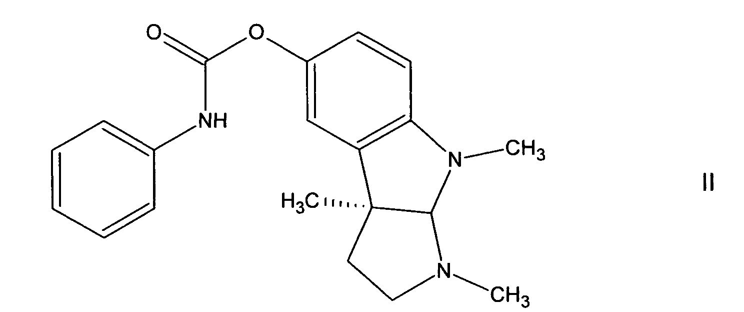 (3a*R*)-1,3a,8-trimethyl-1,2,3,3a,8,8a-hexahydropyrrolo[2,3-*b*]indol-5-yl phenylcarbamate

As used herein, the phrases *"co-administration," "in combination with," "the combination of"* or similar phrases referring to two or more drugs or compounds means that the compounds are present in the subject being treated at the same time. The compounds may be administered at the same time or sequentially in any order at different points in time. However, the compounds should be administered sufficiently closely in time so as to provide the desired enhancement of treatment effect. The compounds may be administered by the same route of administration or by different routes of administration. Suitable dosing intervals, routes and the order of administration with such compounds will be readily apparent to those skilled in the art, in light of the present disclosure.

As used herein, *"(-)-phenserine"* and *phenserine* mean (-)-N-phenylcarbamoyl eseroline (which may also be referred to as: (3aS)-1, 3a, 8-trimethyl-1, 2, 3, 3a, 8, 8a-hexahydropyrrolo[2, 3-b]indol-5-yl phenylcarbamate) and pharmaceutically acceptable salts and esters thereof. Additional (-) carbamates of the present invention include, but are not limited to, (-)-2'-methylphenylcarbamoyleseroline, (-)-2'-4'-dimethylphenylcarbamoyleseroline, (-)-4'-methylphenylarbamoyleseroline, (-)-2'-ethylphenylcarbamoyleseroline, (-)-phenylcarbamoyleseroline, (-)-(-)-2',4',6'-trimethylphenylcarbamoyleseroline, (-)-2'-chlorophenylcarbamoyleseroline, (-)-2',6'-dichlorophenylcarbamoylseroline, (-)-physovenol; (-)-5-O-(2'-methylphenylcarbamoyl)physovenol; (-)-3, 3a, 8, 8a-tetrahydro-3a, 8-dimethyl-2H-thieno-[2,3-b]indole-5-ol butyl carbamate; (-)-3, 3a,8,8a-tetrahydro-3a,8-dimethyl-2H-thieno[2,3-b]indole-5-ol heptylcarbamate; (-)-3,3a,8,8a-tetrahydro-3a,8-dimethyl-2H-thieno[2,3-b]indole-5-ol phenylcarbamate; (-)-3,3a,8,8a-tetrahydro-3a,8-dimethyl;-2H-thieno[2,3-b]indole-5-ol 2'-methylphenylcarbamate; (-)-3,3a,8,8a-tetrahydro-3a,8-dimethy-2H-thieno [2,3-b]indole-5-ol 2'-isopropylphenylcarbamate; (-)-thiaphysovenine, (-)-Phenyl-thiaphysovenine; (-)-2',4'-dimethylphenyl-thiaphysovenine; heptyl-physostigmine and pharmaceutically acceptable salts or esters thereof.

As used herein, *"(+)-phenserine"* and/or *"POSIPHEN™"* means (3a*R*)-1, 3a, 8-trimethyl-1, 2, 3, 3a, 8, 8a-hexahydropyrrolo[2, 3-*b*]indol-5-yl phenylcarbamate, which includes pharmaceutically acceptable salts or esters thereof. In addition, (+)-phenserine is used herein as an example of a compound not having significant AChEI activity, but capable of decreasing β-APP synthesis and/or production or accumulation of Aβ, such compounds include (+)-tolserine, (+)-N¹-benzylnorphysostigmine, (+) N¹-benzylnorphenserine, (+) N¹-enzylnortolserine, (+)-N¹-benzylnorcymserine, (+)-N¹-phenethylnorcymserine, (+)-cymserine, (+)-N¹-norphenserine, (+) N¹-nortolserine, (+) N¹-norcymserine, (+)-N⁸-benzylnorphenserine, (+)-N⁸-norphenserine, (+)-N¹, N⁸-bisbenzylnorphenserine, (+)-N¹, N⁸-bisnorphenserine, (+) N¹, N⁸-isnorcymserine, compounds shown in WO 02/48150, and pharmaceutically acceptable salts or esters thereof. As will be recognized by a person of ordinary skill in the art (+)-phenserine provides an example of the desired compound, enabling the person of skill in the art to utilize alternative compounds.

As used herein, reference to a compound, for example, (3a*R*)-1, 3a, 8-trimethyl-1, 2, 3, 3a, 8, 8a-hexahydropyrrolo[2, 3-*b*]indol-5-yl phenylcarbamate, (-)-phenserine, donepezil, metrifonate, tacrine and rivastigmine, will be understood to include pharmaceutically acceptable salts and esters thereof, whether or not such reference to a compound is expressly followed by reference to such salts or esters.

Phenserine is a carbamate analog of physostigmine (Phy), which is a long-acting inhibitor of cholinesterase. Phenserine was first prepared by Polonovski, (1916) *Bull. Soc. Chim.* 19:46-59, and technical details were summarized by Beilstein, *Handbuch der Organischen Chemie,* 4th ed. Vol. 23. Springer Verlag, Berlin, pp 333 (1954)). (-)-Phenserine not only inhibits the degradation of the neurotransmitter acetylcholine, it inhibits the production of a toxic form of the β-amyloid precursor protein (β-APP). U.S. Patents 5,171,750 and 5,998,460 disclose the use of substituted (-)-phenserine compounds as anticholinesterase (AChE) inhibitors for the treatment of cholinergic disorders such as glaucoma, Myasthenia Gravis, Alzheimer's disease and as an antidote against poisoning with organophosphates.

Despite the fact that (-)-phenserine decreases the levels of extracellular β-APP in the CSF of adult rats, the decrease reaches a plateau that is not further decreased even at very high doses. Further, the concentration of (-)-phenserine used to provide a desirable cholinergic effect in a human may not optimally decrease β-APP and Aβ levels. Thus, providing a subject with an amount of (-)-phenserine optimally effective in the reduction of β-APP synthesis may produce undesirable overstimulation of the cholinergic system.

The compound of formula II has been disclosed by Pei, Greig, *et al*. Article entitled "Inhibition of Human Acetylcholinesterase" *Med. Chem. Research Acad.* (1995) 5:265-270. In this article, it was shown that unlike its negative enantiomer, (+)-phenserine, the compound of formula II, was far less active as an inhibitor of human acetylcholinesterase. However, despite the absence of significant inhibition of acetylcholinesterase, the compound of formula II has a surprisingly potent effect in the reduction of the levels of the potentially toxic amyloid-β peptide (Aβ) (FIG. 5).

The Aβ protein is associated with a progressive neurodegenerative condition leading to loss of memory characterized by the appearance of senile plaques that are primarily composed of an Aβ and neurofibrillary tangle aggregates (for example, *see,* Shaw *et al.* (2001) (-)-phenserine Regulates Translation of β-amyloid Precursor protein mRNA by a Putative Interleukin-1 Responsive Element, a Target for Drug Development, *Proc. Natl. Acad. Sci. USA* 98(13):7605-7610).

The Aβ protein is a 40- to 42-residue peptide derived from the larger protein β-APP, which contains 695-770 residues. β-APP is believed to be cleaved by β-and γ-secretases to generate the β-amyloid protein (Aβ), which is an important step in the pathogenesis of AD (*see,* Caswell *et al.,* (1999) The Amyloid B-Protein Precursor of Alzheimer's Disease is Degraded Extracellularly by a Kunitz Protease Inhibitor Domain-Sensitive Trypsin-Like Serine Protease in Cultures of Chick Sympathetic Neurons, *Eur. J. Biochem.* 266:509-516).

The IC₅₀ for inhibition of β-APP synthesis may be assayed by the method of Ying *et al*., (1989) Measurement of inhibition of protein biosynthesis in a rabbit reticulocyte lysate, Progress in biochemistry and Biophysics; 16(6):467-470.

Not all AChEI reduce β-APP or Aβ production. For example, donepezil (1.5 mg/kg *o.p*.) increased extracellular β-APP in adult rats (FIG. 1) and decreased intracellular levels (FIG. 2), but only decreased Aβ levels when administered at a dosage sufficient to cause cellular toxicity (FIG. 2). In addition, daily administration of metrifonate (80 mg/kg) or tacrine (3 mg/kg), administered for three weeks, did not alter secreted β-APP levels in adult rats (data not shown) and rivastigmine (50 µM) elevates the levels of secreted β-APP, without altering Aβ levels (FIG. 3). In contrast, (-)-phenserine reduces β-APP and Aβ levels in a concentration-dependent manner (FIG. 4). Thus, not all AChEIs reduce β-APP levels, either intracellular or extracellular, or reduce Aβ levels. Therefore, an exemplary embodiment of the invention relates to the use of (+)-phenserine in combination with an AChEI selected from the group consisting of at least one of (-)-phenserine, donepezil, metrifonate, tacrine, galantamine and rivastigmine.

(-)-Phenserine and (+)-phenserine, reduce the synthesis of β-APP, for example, to a level that retains its physiological function, but reduces the β-amyloid protein (Aβ) derived from it, thereby lowering β-amyloid levels without affecting β-APP proteolytic enzymes, which are reported to possess other critical functions. Reducing β-amyloid formation and its resulting deposition may be used to favorably modify AD progression (Lahiri *et al.* (2000) Cholinesterase Inhibitors, Beta-amyloid Precursor Protein and Amyloid Beta-peptides in Alzheimer's Disease, *Acta Neurol. Scand. Suppl.* 176:60-67). Hence, the invention provides the surprising ability to use a mixture of (-)-phenserine and (+)-phenserine for the treatment of cognitive disorders. Therefore, the invention provides a method of treating or prophylaxing a subject without the necessity of incurring the additional costs associated with purification of the desired enantiomer.

The present invention discloses the different inhibition parameters affecting AChE inhibition and β-APP reduction, thereby providing a method for enhancing treatment of cognitive disorders. The present invention demonstrates the difference in inhibition (IC₅₀) concentrations for each enantiomer on both the production of β-APP and the cholinergic effect. Without the guidance of the present invention, a person of ordinary skill in the art would not be motivated to co-administer both an AChEI (*e.g.*, (-)-phenserine) and (+)-phenserine to a subject having or believed to be susceptible to a cognitive disorder). Particularly, in light of the fact that (-)-phenserine is a strong AChEI, whereas (+)-phenserine is not.

The IC₅₀ values can be determined for the compounds of the invention using methods well known in the art (*see,* Greig *et al.* (2000) The Experimental Alzheimer Drug phenserine: Preclinical Pharmacokinetics and Pharmacodynamics, *Acta Neurol. Scand. Suppl.* 176:74-84). For example, the pharmacological activity of each compound may be expressed as the IC₅₀, which is defined as the concentration (*e.g.,* in nanomoles) required to inhibit 50% of the enzyme activity of AChE or produce a 50% reduction in β-APP or Aβ. The AChE inhibition IC₅₀ values may be determined by measuring the enzyme activity at each concentration and expressed as a percentage of the activity determined in the absence of each compound. This then is transformed into a logit format, where logit = In (% activity/ [100 - % activity]), and was plotted as a function of the log concentration of the compound (*Id*.). IC₅₀ values (*i.e.,* logit = ln (50/ [100-50] = 0) were determined only from correlation coefficients (r²) of less than -0.985 and when more than 50% inhibition was achieved from duplicate samples.

A combination of both an AChEI and (+)-phenserine may be used to produce a particularly desirable treatment for cognitive disorders. For example, administration of (-)-phenserine, an example of an AChEI, at an optimal dose for the reduction of β-APP may produce undesirable side effects, due to its high AChEI activity. For example, at such a concentration the cholinergic effect of (-)-phenserine may result in cholinergic over stimulation. The present invention makes use of the different inhibition concentrations (IC₅₀) for each drug in relation to effects on anticholinesterase activity and anti-β-APP activity.

For example, the IC₅₀ of (-)-phenserine on acetylcholinesterase (AChE) is about 22 nM. In contrast, the IC₅₀ of (+)-phenserine on AChE is greater than 25,000 nM. Thus, (+)-phenserine provides little or no AChE inhibition (AChEI).

| IC₅₀ Value (nM) | | | |
|---|---|---|---|
| Compound | AChE | BChE | Selectivity |
| (-)-Physostigmine (RBC/plasma) | 28 ± 2 | 16 ± 3 | 2-fold BChE |
| (-)-Physostigmine (Brain) | 31 ± 8 | 130 ± 55 | 4-fold AChE |
| (-)-Phenserine (RBC/plasma) | 22 ± 1 | 1560 ± 270 | 70-fold AChE |
| (-)-Phenserine (Brain) | 36 ± 3 | 2500 ±1100 | 70-fold AChE |

IC₅₀ values were determined in duplicate on a minimum of three different occasions.

In contrast to the potent inhibition of AChE by (-)-phenserine, the IC₅₀ for inhibition of β-APP is about 1.1 µM and about 670 nM for intracellular and extracellular β-APP levels, respectively. Thus, (-)-phenserine is between approximately 30 and 50 times more effective in the inhibition of AChE than it is in reducing β-APP levels. Hence, (+)-phenserine may be coadministered with (-)-phenserine at a ratio of between about 2:1 to about 100:1, preferably between about 10:1 to about 70:1, or between about 30:1 to about 50:1, where the ratio is the molar ratio of each active compound. Thus, providing a level of (-)-phenserine, (an example of an AChEI) sufficient to effectively inhibity AChE activity and (+)-phenserine at a level sufficient to effectively inhibit secretion of β-APP.

| Compound | IC₅₀ for AChE inhibition | IC₅₀ for β-APP secretion | IC₅₀ for intracellular β-APP |
|---|---|---|---|
| (-)-phenserine | ≈ 22 nM^{†} | ≈ 670 nM | ≈ 1,100 nM |
| (+)-phenserine | > 25,000 nM | ≈ 670 nM | ≈ 1,100 nM |

| | | | |
|---|---|---|---|
| ^{†} *See,* al-Jafari *et al.* (1998) Kinetics of Human Erythrocyte Acetylcholinesterase Inhibition by a Novel Derivative of Physostigmine: (-)-phenserine, *Biochem. Biophys. Res. Commun.* 248(1):180-185. | | | |

In an exemplary embodiment, the invention provides a dosage of (-)-phenserine of about 5 mg, about 10 mg, about 15 mg, about 20 mg, about 25 mg, and/or about 30 mg *bid*. Additional dosages for (-)-phenserine are disclosed in U.S. Patent 5,409,948. The (-)-phenserine may be co-administered with about 50 mg, about 100 mg, 150 mg, about 200 mg, about 250 mg, about 300 mg, about 350 mg, about 400 mg, about 450 mg, or about 500 mg of (+)-phenserine. By combining the AChEI activity of (-)-phenserine with (+)-phenserine, β-APP and/or Aβ levels can be decreased, without inducing undesirable side effects associated with cholinergic overstimulation. For example, (+)-phenserine may be co-administered with (-)-phenserine in a ratio of about 1:1, about 2:1, about 3:1, about 4:1, about 5:1, about 6:1, about 7:1, about 8:1, about 9:1, about 10:1, about 15:1, about 20:1, about 25:1, about 30:1, about 35:1, about 40:1, about 45:1, about 50:1, about 55:1, about 60:1, about 65: 1, about 70: 1, about 75: 1, about 80:1, about 85: 1, about 90:1, about 95:1 and/or about 100:1 (wt/wt), where the weight is based on the weight of the active compound.

(+)-Phenserine may also be coadministered with (-)-phenserine or other acetylcholinesterase inhibitors in non-equal molar amounts, for example about 2:1, about 3:1, about 4:1, about 5:1, about 6:1, about 7:1, about 8:1, about 9:1, about 10:1, about 15:1, about 20:1, about 21:1, about 22:1, about 23:1, about 24:1, about 25:1, about 26:1, about 27:1, about 28:1, about 29:1, about 30:1, about 31:1, about 32:1, about 33:1, about 34:1, about 35:1, about 36:1, about 37:1, about 38:1, about 39:1, about 40:1, about 45:1, about 50:1, about 55:1, about 60:1, about 65:1, about 70:1, about 75:1, about 80:1, about 85:1, about 90:1, about 95:1 and/or about 100:1, wherein the ratio is expressed as the number of moles of each active compound. In an exemplary embodiment, the invention relates to a ratio of (-)-phenserine to (+)-phenserine that is not at a ratio of about 1:1.

Donepezil, e.g., donepezil hydrochloride, may be administered in dosage forms containing, for example, 5 or 10 mg donepezil. Inactive ingredients, such as lactose monohydrate, corn starch, microcrystalline cellulose, hydroxypropyl cellulose, and magnesium stearate, may be added as desired. Tacrine may be administered in dosage of, for example, 20, 40 (*e.g*., 10 mg 4 times daily), or 80 mg of tacrine daily, up to about 160 mg daily (*e.g*., 40 mg 4 times daily). Metrifonate may be administered in dosages of, for example, about 10 to about 100 mg, or about 30 to about 80 mg per day. Rivastigmine may be administered in dosages of, for example, about 2 to about 20 mg, or about 6 to about 12 mg, per day. Dosages are preferably determined by persons of ordinary skill in the art and may be modified based on a large number of factors commonly considered by persons of ordinary skill in the art, for example, physicians.

In another exemplary embodiment, (+)-phenserine may be co-administered with an acetylcholinesterase inhibitor selected from the group consisting of (-)-phenserine, donepezil, rivastigmine, metrifonate, tacrine, physostigmine, (-) carbamates (*e.g*., those listed herein), eptastigmine, galantamine, and huperzine A.

In another exemplary embodiment, (+)-phenserine may be coadministered with an AChEI, such as (-)-phenserine, allowing the dosage of the AChEI to be decreased, thereby reducing undesirable side effects, while maintaining a beneficial affect on β-APP or Aβ levels.

In another exemplary embodiment, (+)-phenserine may be administered to a subject thought to be at risk for the development of a cognitive impairment associated with increased levels of β-APP or Aβ, thereby reducing the subject's risk of developing a cognitive impairment and/or providing a prophylactic treatment.

The ability of the compounds of the present invention, and/or one or more salts or esters, to improve cognitive performance can be assessed by various methods known in the art. For example, standard tests for measuring the progression of a cognitive disease state include, but are not limited to, the Mini-Mental State Examination (MMSE), Clinical Dementia Rating, Global Deterioration Scale (GDS) and the Alzheimer's Disease Assessment Scale (ADAS-cog). The ADAS-cog is a multi-item instrument for measuring cognitive performance which includes elements of memory, orientation, retention, reasoning, language and praxis. The ADAS-cog scoring ranges from 0 to 70 with higher scores indicating cognitive impairment. Elderly normal adults may score as low as 0 to 1, but it is not unusual for non demented adults to score more highly. In measuring by the ADAS-cog test, one measures the changes over an extended period of time before and during treatment to determine the progression of this disease and also to compare this rating with untreated subjects. Subjects treated in accordance with the method of this invention may be found to have, on average, better scores under this test as compared to untreated subjects.

The ability of the method of the invention can also be assessed using a Clinician's Interview Based Impression of Change plus use of caregiver information, the CIBIC-Plus. The CIBIC-Plus is not a single instrument and is not a standardized instrument like the ADAS-cog. Clinical trials for investigational drugs have used a variety of CIBIC formats, each different in terms of depth and structure. As such, results from a CIBIC-Plus reflect clinical experience from the trial or trials in which it is used and can not be compared directly with the results of CIBIC-Plus evaluations from other clinical trials. It may be formulated to represent an assessment of a skilled clinician, using validated scales based on the clinician's observations at interviews with the subject and a caregiver familiar with the behavior of the subject over the interval rated. The CIBIC-Plus is scored as a seven point categorical rating, ranging from a score of 1, indicating "markedly improved," to a score of 4, indicating "no change," to a score of 7, indicating "marked worsening."

The ability of the compounds of the present invention, and/or one or more salts or esters, to reduce the rate of Aβ peptide accumulation can be assessed by various methods known in the art. For example, by direct measurement of peptide levels, such as with an ELISA assay, and/or indirectly through a cognitive test.

It is important to note that any treatment for cognitive disorders such as Alzheimer's disease and other age-related cognitive impairments require chronic treatment (*i.e*., continuous treatment) throughout the life of the subject. In this manner, the deterioration due to cognitive impairment, and the symptoms of such cognitive impairment, are stabilized or ameliorated and in some cases improved. The cognitive disorders which result from diseases such as Alzheimer's disease are progressive throughout the life of the subject. Therefore, the method of this invention provides a means for reducing the progression of these disease states, for example, by additional reductions in β-APP and/or Aβ.

The dosage for treatment typically depends upon the route of administration, the age, weight and condition with regard to cognitive impairment of the subject to be treated. In general, dosages of (3aR)-1,3a,8-trimethyl-1,2,3,3a,8,8a -hexahydropyrrolo[2,3-b]indol-5-yl phenylcarbamate and/or its salts are disclosed in PCT International Publication WO 03/082270A1 and dosages of (-)-phenserine and/or its salts are disclosed in U.S. Patents 5,171,750 and 5,998,460, all of which are hereby incorporated by reference. Based on the information presented herein, determination of the effective amount of a drug is well within the skill of the ordinary practitioner in the art. The compositions are generally useful in pharmaceutical compositions (wt%) of the active ingredients with a carrier or vehicle in about 0.1 to 99 wt % and preferably about 25-85 wt %.

Methods of manufacturing pharmaceutical dosage forms, such as tablets, capsules, and parenteral vehicles, are well known in the art. One or more compounds of this invention may be utilized in compositions such as tablets, capsules or elixirs for oral administration, suppositories, sterile solutions or suspensions or injectable administration, and the like. A person of ordinary skill in the art may prepare formulations of the compounds of this invention for storage or administration by mixing the compound or compounds having a desired degree of purity with physiologically acceptable carriers, excipients, stabilizers etc. Acceptable carriers or diluents for therapeutic use are well known in the pharmaceutical field, and are described, for example, in *Remington's Pharmaceutical Sciences,* Mack Publishing Co., (A. R. Gennaro edit. 1985), the contents of which are incorporated by this reference. Such materials are nontoxic to the recipients at the dosages and concentrations employed, and include buffers such as phosphate, citrate, acetate and other organic acid salts, antioxidants such as ascorbic acid, low molecular weight (less than about ten residues) peptides such as polyarginine, proteins, such as serum albumin, gelatin, or immunoglobulins, hydrophilic polymers such as polyvinylpyrrolidone, amino acids such as glycine, glutamic acid, aspartic acid, or arginine, monosaccharides, disaccharides, and other carbohydrates including cellulose or its derivatives, glucose, mannose or dextrins, chelating agents such as EDTA, sugar alcohols such as mannitol or sorbitol, counterions such as sodium and/or nonionic surfactants such as Tween, Pluronics or polyethyleneglycol. Sterile compositions for injection can be formulated according to conventional pharmaceutical practice. For example, dissolution or suspension of the active compound in a vehicle such as an oil or a synthetic fatty vehicle like ethyl oleate, or into a liposome may be desired. Buffers, preservatives, antioxidants and the like can be incorporated according to accepted pharmaceutical practice.

A composition of the invention may include an effective amount of (+)-phenserine and (-)-phenserine. The amount of active compound that can be combined with a carrier material to produce a single dosage form will vary depending upon the disease treated, the mammalian species, and the particular mode of administration. However, as a general guide, suitable unit doses for the compounds of the present invention can, for example, preferably contain between about 0.001 grams to about 1 gram of (-)-phenserine, for example, about 5 mg, about 7.5 mg, about 10 mg, about 15 mg, about 20 mg, or about 30 mg, and/or between about 0.001 g to about 1g or about 5 mg, 10 mg, about 20 mg, up to about 1g of (3aR)-1,3a,8-trimethyl-1,2,3,3a,8,8a-hexahydropyrrolo[2,3-*b*]indol-5-yl phenylcarbamate. Typically, the active compound, as the free acid or base form or as a pharmaceutically acceptable salt (e.g., the tartrate salt), is compounded with a physiologically acceptable vehicle, carrier, excipient, binder, preservative, stabilizer, dye, flavor etc., as called for by accepted pharmaceutical practice. For example, a capsule or tablet containing, for example, 5 mg, 10 mg or 15 mg of (-)-phenserine may be made by methods well known in the art by admixing an acceptable carrier, excipient, preservative, stabilizer or the like. Similarly, a capsule or tablet containing (3aR)-1,3a,8-trimethyl-1,2,3,3a,8,8a-hexahydropyrrolo[2,3-b]indol-5-yl phenylcarbamate may be made by methods well known in the art.

The term "pharmaceutically acceptable salts" includes acid addition salts, which are any non-toxic organic or inorganic acid addition salt. Illustrative inorganic acids which form suitable salts include hydrochloric, hydrobromic, sulfuric, and phosphoric acid and acid metal salts such as sodium monohydrogen orthophosphate, and potassium hydrogen sulfate. Illustrative organic acids which form suitable salts include the mono-, di-, and tricarboxylic acids. Illustrative of such acids are, for example, acetic, glycolic, lactic, pyruvic, malonic, succinic, glutaric, fumaric, malic, tartaric, citric, ascorbic, maleic, hydroxymaleic, benzoic, hydroxybenzoic, phenylacetic, cinnamic, salicylic, 2-phyenoxybenzoic, and sulfonic acids such as p-toluene sulfonic acid, methanesulfonic acid and 2-hydroxyethanesulfonic acid. Preferred salts include tartrate, phosphate, and fumarate. In addition, esters, where desirable and appropriate, are included within the scope of pharmaceutically acceptable salts.

The invention is further explained by way of the following illustrative Examples.

### Example I

Not all AChEI reduce β-APP or Aβ production (compare, Inestrosa *et al.* (1996) Acetylcholinesterase accelerates assembly of amyloid-beta-peptides into Alzheimer's fibrils: possible role of the peripheral site of the enzyme, *Neuron* 16(4):881-91; and Inestrosa *et al.* (1996) Acetylcholinesterase is a senile plaque component that promotes assembly of amyloid beta-peptide into Alzheimer's filaments, *Mol. Psychiatry* 1(5):359-61). For example, donepezil (1.5 mg/kg *o.p*.) increased extracellular β-APP in adult rats (FIG. 1) and decreased intracellular levels (FIG. 2), but only decrease Aβ levels when administered at a dosage sufficient to cause cellular toxicity (FIG. 2). In addition, daily administration of Metrifonate (80 mg/kg) or Tacrine (3 mg/kg), administered for three weeks, did not alter secreted β-APP levels in adult rats (data not shown) and rivastigmine (50 µM) elevates the levels of secreted β-APP, without a statistically significant change Aβ levels (FIG. 3). In contrast, (-)-phenserine reduces β-APP and Aβ levels in a concentration-dependent manner (FIG. 4). Thus, not all AChEIs reduce β-APP levels, either intracellular or extracellular), or reduce Aβ levels.

### Example II

(3aR)-1, 3a, 8-trimethyl-1, 2, 3, 3a, 8, 8a-hexahydropyrrolo[2, 3-b]indol-5-yl phenylcarbamate was tested in parallel with (-)-phenserine for controlling β-APP levels by the procedure disclosed in the Shaw *et al.* (2001), *Proc. Nat. Acad. Sci. USA* 98 (13):7605-7610. The methodology of Shaw *et al*. for carrying out these tests is summarized as follows:

SK-N-SH neuroblastoma cells were cultured on 60 mm dishes at a concentration of 3 x 10⁶ cells, and SH-SY-5Y neuroblastoma cells were plated in 100 mm dishes at a concentration of 3 x 10⁵ cells. The cells were allowed to grow in complete media (10% FBS, 2 mM glutamine in DMEM) for 3 to 4 days until they reached 70% confluence. To start the experiment, spent media was removed and replaced with fresh media (DMEM+0.5% FBS) containing 0, 5 or 50 µM of either (-)-phenserine or (3aR)-1, 3a, 8-trimethyl-1, 2, 3, 3a, 8, 8a-hexahydropyrrolo [2,3-b]indol-5-yl phenylcarbamate, and cells were incubated at 37 °C, 5% CO₂ for the specific times indicated.

Lysate preparation: At each time point, the spent medium was collected and stored at -70 C for later analysis of secretory β-APP levels. Cell lysates were prepared as reported previously (Lahiri *et al.* (1997) Effects of Cholinesterase Inhibitors on the Secretion of Beta-Amyloid Precursor Protein in Cell Cultures, *Ann. N.Y. Acad. Sci.* 826:416-421; and Lahiri *et al.* (1998) The Secretion of Amyloid Beta-Peptides is Inhibited in the Tacrine-Treated Human Neuroblastoma Cells, *Mol. Brain Res.* 62:131-140). Protein levels of the supernatant were analyzed by the Bradford protein assay (BioRad, Mellville, NY).

Western Blot: Fifteen µg of protein from each sample was loaded onto a 10% NuPAGE Bis-Tris gel in 1X NuPAGE MOPS SDS running buffer (NOVEX, San Diego, CA) and the proteins separated at 200 V for 45 minutes. The gels were then transferred onto nitrocellulose at 25 V for 1.5 hours. Non-specific binding was blocked, and each blot was probed for 2 hours with either 22C11 anti-β-APP N-terminal antibody (2.5 µg/mL, Boehringer Mannheim, Indianapolis, IN) or anti-activated ERK antibody (25 ng/mL, Promega, Madison, WI). Anti-mouse IgG- or anti-rabbit IgG, conjugated to horse radish peroxidase, were used as secondary antibodies. Equivalent loading of samples was determined by Ponceau S staining (Sigma, St. Louis, MO). Densimetric quantification of the chemiluminescence of blots was undertaken by using a CD camera and NIH-IMAGE (version 4.1).

Lactate Dehydrogenase Assay: Measurement of released lactate dehydrogenase (LDH) in the conditioned medium was undertaken as a marker of cell viability and integrity, as described previously (Lahiri *et al.,* 1997 and 1998).

Total Aβ Assay: Total Aβ peptide levels in SH-SY-5Y and SK-N-SH cultured samples were assayed by a sensitive ELISA assay (Suzuki *et al.,* (1994) An Increased Percentage of Long Amyloid Beta Protein Secreted by Familial Amyloid Beta Protein Precursor (beta APP717) Mutants, *Science* 264:1336-1340). For total Aβ peptide measurements, a sandwich immunoassay with rabbit polyclonal antibody to residues 1-40 of Aβ or a monoclonal antibody to residues 17-25 of Aβ as a capture antibody for all species of Aβ peptide, Aβ1-40 and Aβ1-42, was used, and the values were expressed as the mean of six independent assays.

The results of this test demonstrate a decrease in β-APP levels, relative to controls, at various time intervals utilizing (-)-phenserine or (3aR)-1, 3a, 8-trimethyl-1, 2, 3, 3a, 8, 8a-hexahydropyrrolo[2, 3-b]indol-5-yl phenylcarbamate at dosages of the 0.5 µM to 50 µM. As seen in FIG. 4, when compared to the control, the use of high dosages of (-)-phenserine decreased the β-APP levels in the SK-N-SH cells. Even after 16 hours the amount of β-APP protein was reduced by the use of (-)-phenserine. FIG. 4 also demonstrates that the levels of Aβ protein were substantially reduced from that of the controls especially at 16 hours through the use of (-)-phenserine. FIG. 5 compares (3aR)-1, 3a, 8-trimethyl-1, 2, 3, 3a, 8, 8a-hexahydropyrrolo[2,3-b]indol-5-yl phenylcarbamate ((+)-Phenserine) with (-)-phenserine, and as seen from this graph, both the compounds are capable of reducing the β-APP and Aβ protein levels as compared to the control. Therefore, (3aR)-1, 3a, 8-trimethyl-1, 2, 3, 3a, 8, 8a-hexahydropyrrolo[2,3-b]indol-5-yl phenylcarbamate, which lacks anticholinesterase activity, has similar action on the β-APP and Aβ proteins as (-)-phenserine and may be combined with (-)-phenserine or other AChEI compounds without inducing cholinergic overstimulation.

### Example III

One or more compounds lacking or having reduced AChEI activity, for example, one or more compounds disclosed in WO 02/48150 capable of reducing production of β-APP or Aβ is tested in parallel with one or more compounds having AChEI activity, such as (-)-phenserine, donepezil, rivastigmine, metrifonate, physostigmine, (-) carbamates, eptastigmine, galantamine, and huperzine A, for controlling β-APP levels. The compounds may be tested by the procedure utilized in Example II.

(+)-Phenserine is found to reduce the production of β-APP when coadministered with donepezil.

(+)-Phenserine is found to reduce the production of β-APP when coadministered with rivastigmine.

(+)-Phenserine is found to reduce the production of β-APP when coadministered with eptastigmine.

(+)-Phenserine is found to reduce the production of β-APP when coadministered with galantamine.

(+)-Phenserine is found to reduce the production of β-APP when coadministered with huperzine A.

(+)-Phenserine and donepezil, rivastigmine, eptastigmine, galantamine, and/or huperzine A are tested essentially as described in Example IV using an appropriate dosage of the AChEI, *e.g*., a clinically effective amount. The dosage of (+)-phenserine may be adjusted in accordance to the relevant parameters, which will be understood by a person of ordinary skill in the art in light of the present specification (for example, using the IC₅₀ for β-APP inhibition).

### Example IV

On recent administration of (-)-phenserine to rodents by the i.p. route (1ml/kg in isotonic saline) a fine tremor was observed at a dose of 5 mg/kg, which persisted for about 1 hour (*see*, PCT International Publication WO 03/082270). This is a classical central (*i.e.,* brain) cholinergic over-stimulation (overdrive) effect. Tremor, together with symptoms of peripheral over-stimulation (specifically, lacrimation and salivation), were seen at a dose of 7.5 mg/kg (-)-phenserine. At a dose of 20 mg/kg (-)-phenserine rodents are incapacitated by severe tremor and peripheral side effects (particularly salivation: making breathing difficult), and of 5 treated animals 2 were killed when moribund. However, when the same 20 mg/kg dose is given as (+)-phenserine, animals were entirely without symptoms (even minor tremor and appeared similar to both vehicle treated untreated animals). Thus, (3aR)-1, 3a, 8-trimethyl-1, 2, 3, 3a, 8, 8a-hexahydropyrrolo[2, 3-b]indol-5-yl phenylcarbamate may be administered in significantly higher dosages than (-)-phenserine.

(-)-Phenserine improves learning and performance in rodents (as well as in man) via its action as an anticholinesterase, which elevates levels of the cholinergic neurotransmitter, acetylcholine; which is depleted in the Alzheimer brain. The neurotransmitter, acetylcholine has numerous functions outside the brain, controlling heart rate (via the vagus nerve), gastric motility, sweating, salivation, lacrimation, etc. It is stimulation of these actions, as well as over-stimulation of the brain cholinergic system, that results in the toxicity of classical anticholinesterases (e.g., the anticholinesterase drugs: rivastigmain and galanthamine). On the other hand, (3aR)-I, 3a, 8-trimethyl-1, 2, 3, 3a, 8, 8a-hexahydropyrrolo[2, 3-b]indol-5-yl phenylcarbamate lacks anticholinesterase activity and hence lacks cholinergic action. It can therefore be administered in higher amounts than (-)-phenserine, thereby allowing it to be combined with an AChEI at a dosage effective for reducing or further reducing β-APP and/or Aβ levels.

### Example V

Relatively high doses of (-)-phenserine reduce β-APP levels in the CSF of naive rats. FIG. 6 illustrates the reduction in β-APP levels by administering 2.5 mg/kg of (-)-phenserine.

FIG. 7 illustrates the maximum AChE inhibition of 1 mg/kg (-)-phenserine. A maximum inhibition of 73.5% at 5 minutes was achieved and the inhibition maintained with a slow decay throughout the study, declining to 43.0% inhibition at 8 hours. In this experiment, three-month-old male Fischer-344 rats were anesthetized with halothane. PE 50 catheters, filled with heparinized isotonic saline, were tied into their right femoral artery and vein. Animals then were restrained with a plaster cast that enabled them to move their head and forequarters only and were allowed to recover from anesthesia in a temperature-controlled enclosure. Samples of plasma were removed to measure untreated levels of ACHE activity. At 90 minutes after surgery, hexamethonium bromide (5 mg/kg) was administered intraperitoneally, followed by subcutaneous injection of atropine methyl bromide (4 mg/kg) 10 minutes later. These quaternary nicotinic and muscarinic blocking agents, respectively, do not cross the blood brain barrier and inhibit peripheral cholinergic overdrive, caused by AChE inhibition, which otherwise would be deleterious. At 2 hours after surgery, either 2 mg/kg of physostigmine or 1 mg/kg of (-)-phenserine was administered intravenously. Both were formulated in a manner consistent for intravenous bolus injection. Plasma samples were removed and immediately frozen at -70 °C., just prior to intravenous administration of the inhibitors and at times between 2 minutes and 8 hours.

### Example VI

This example shows a method by which efficacy of a racemic mixture of formulas I and II, for example, as a tartrate salts, can be measured.

A randomized, double-blind, placebo-controlled study is done to measure the efficacy of the mixture of (+) and (-) enantiomers of phenserine given daily over twelve (12) weeks. Sixty subjects diagnosed as having mild to moderate Alzheimer's disease are enrolled in a study program. The subjects may constitute male and female subjects between the ages of about 50 and 85 years.

Forty subjects receive an oral dose of 5 mg *bid* of (-)-phenserine and 50 mg *bid* of (3aR)-1,3a,8-trimethyl-1,2,3,3a,8,8a-hexahydropyrrolo[2,3-b]indol-5-yl phenylcarbamate ((+)-phenserine). Concurrently, twenty subjects assigned to placebo medication receive matched placebo capsules for the entire 12 week duration of the study. A sufficient number of potential subjects are screened to ensure enrollment of 60 eligible cases.

All study participants are evaluated prior to the study (First Level) and periodically throughout using one or more of the following standard efficacy tests:
- NPI (Neuropsychiatric Inventory;
- CGIC (Clinician's Global Impression of Change);
- ADAS-cog (Alzheimer's Disease Assessment Scale - cognitive subscale);
- MMSE (Mini-Mental State Exam);
- CANTAB (Cambridge Neuropsychological Test Automated Battery); and
- ADCS-ADL (Activities of Daily Living).

At the end of the twelve-week test, the subjects in the treated group maintain a level at least as great as the First Level prior to treatment with respect to some or all of the above tests. Some of the subjects demonstrate an improvement in this level at the end of the twelve-week period. On the other hand, with respect to the untreated subjects there is no improvement over the First Level as measured by the above tests and most of the subjects in this control group show a decline from the First Level.

### Example VII

In preclinical studies, (-)-phenserine reduced Aβ levels in nerve cells by 14% in a rat brain after 8 hours and 31 % after 16 hours, versus controls. (-)-phenserine similarly reduced levels of β-APP, the precursor of beta-amyloid. By combining (3aR)-1, 3a, 8-trimethyl-1, 2, 3, 3a, 8, 8a-hexahydropyrrolo[2, 3-b]indol-5-yl phenylcarbamate with (-)-phenserine Aβ levels are further reduced.

### Example VIII

A randomized, double-blind, placebo-controlled study is done to measure the efficacy of the mixture of (3aR)-1,3a,8-trimethyl-1,2,3,3a,8,8a-hexahydropyrrolo [2,3-b]indol-5-yl phenylcarbamate and a drug, such as, (-)-phenserine, donepezil, tacrine or rivastigmine, given daily over twelve (12) weeks. About sixty subjects diagnosed as having mild to moderate Alzheimer's disease are enrolled in a study program. The subjects may constitute male and female subjects between the ages of about 50 and 85 years.

Forty subjects receive an oral dose of 6-12 mg per day of rivastigmine and 50 mg *bid* of (3aR)-1,3a,8-trimethyl-1,2,3,3a,8,8a-hexahydropyrrolo [2,3-b]indol-5-yl phenylcarbamate. Concurrently, twenty subjects assigned to placebo medication receive matched placebo capsules for the entire 12 week duration of the study. A sufficient number of potential subjects are screened to ensure enrollment of about 60 eligible cases.

All study participants are evaluated prior to the study (First Level) and periodically throughout using one or more of the following standard efficacy tests:
- NPI (Neuropsychiatric Inventory;
- CGIC (Clinician's Global Impression of Change);
- ADAS-cog (Alzheimer's Disease Assessment Scale - cognitive subscale);
- MMSE (Mini-Mental State Exam);
- CANTAB (Cambridge Neuropsychological Test Automated Battery); and
- ADCS-ADL (Activities of Daily Living).

At the end of the twelve-week test, the subjects in the treated group maintain a level at least as great as the First Level prior to treatment with respect to some or all of the above tests. Some of the subjects demonstrate an improvement in this level at the end of the twelve-week period. On the other hand, with respect to the untreated subjects there is no significant improvement over the First Level as measured by the above tests and most of the subjects in this control group show a decline from the First Level. The effects may also be monitored or determined using other testing methods known in the art, for example, *see* Stefanova *et al.* (2003) *Neurosci. Lett.* 338:159-163; Nicholas *et al.* (2001) *Int. J Geriatr. Psychiatry* 16:1104-1106; and Basun *et al.* (2002) *Dement. Geriatr. Cogn. Disord.* 14:156-160.

All references, including publications, patents, and patent applications, cited herein are hereby incorporated by reference to the same extent as if each reference were individually and specifically indicated to be incorporated by reference and were set forth in its entirety herein.

While this invention has been described in certain embodiments, the present invention can be further modified within the spirit and scope of this disclosure. This application is therefore intended to cover any variations, uses, or adaptations of the invention using its general principles. Further, this application is intended to cover such departures from the present disclosure as come within known or customary practice in the art to which this invention pertains and which fall within the limits of the appended claims.

## Claims

1. Use of an acetylcholinesterase inhibitor selected from the group consisting of (-)-phenserine, donepezil, rivastigmine, metrifonate, physostigmine, eptastigmine, galantamine, and huperzine A, and a compound that inhibits production of β-APP or inhibits or slows accumulation of Aβ for the manufacture of a medicament for the treatment of cognitive disorders associated with Aβ accumulation.

2. Use of an acetylcholinesterase inhibitor according to claim 1, wherein the compound that inhibits production of β-APP or inhibits or slows accumulation of Aβ is selected from the group consisting of (+)-phenserine, (+)-tolserine, (+)-N¹-benzylnorphysostigmine, (+)-N¹-benzylnorphenserine, (+)-N¹benzylnortolserine, (+)-N¹-benzylnorcymserine, (+)-N¹-phenethylnorcymserine, (+) cymserine, (+) N¹-norphenserine, (+) N¹-nortolserine, (+) N¹-norcymserine, (+)-N⁸-benzylnorphenserine, (+)-N⁸-norphenserine, (+)-N¹, N⁸-bisbenzylnorphenserine, (+)-N¹, N⁸-bisnorphenserine, and (+)-N¹, N⁸-bisnorcymserine.

3. Use of an acetylcholinesterase inhibitor according to claim 1 or claim 2, wherein the acetylcholinesterase inhibitor is (-)-phenserine, and compound that inhibits production of β-APP or inhibits or slows accumulation of Aβ is (+)-phenserine, wherein the ratio of (-)-phenserine and (+)-phenserine is between about 10:1 1 to about 100:1 1 by weight.

4. Use of an acetylcholinesterase inhibitor according to any one of claims 1 to 3, wherein the (-)-phenserine is administered to said subject in an amount of about 5 mg to about 15 mg twice a day.

5. Use of an acetylcholinesterase inhibitor according to claim 3 or claim 4, wherein said (+)-phenserine and said (-)-phenserine are administered in a ratio between about 20:1 and about 40:1.

6. Use of an acetylcholinesterase inhibitor according to any one of claims 3 to 5, wherein the ratio is about 30:1.

7. Use of an acetylcholinesterase inhibitor according to claim 1 or claim 2, wherein the acetylcholinesterase inhibitor is donepezil.

8. Use of an acetylcholinesterase inhibitor according to claim 1 or claim 2, wherein the acetylcholinesterase inhibitor is rivastigmine.

9. Use of an acetylcholinesterase inhibitor and (+)-phenserine according to claim 2, wherein the acetylcholinesterase inhibitor is eptastigmine.

10. Use of an acetylcholinesterase inhibitor and (+)-phenserine according to claim 2, wherein the acetylcholinesterase inhibitor is galantamine.

11. Use of an acetylcholinesterase inhibitor and (+)-phenserine according to claim 2, wherein the acetylcholinesterase inhibitor is huperzine A.

12. Use of (+)-phenserine for the manufacture of a medicament for the prophylactic treatment of cognitive disorders associated with Aβ accumulation.
